# EUROPEAN PATENT SPECIFICATION

(11) **EP 4 005 501 B1**
(45) Date of publication and mention of the grant of the patent: **24.01.2024**
(21) Application number: 21825366.4
(22) Date of filing: 23.04.2021
(51) Int. Cl.: A61B 17/00, A61B 17/12

(54) **HEAD END ENVELOPING-TYPE CLAMPING AND CONVEYING SYSTEM**
EIN KOPFENDE UMHÜLLENDES KLEMM- UND FÖRDERSYSTEM
SYSTÈME DE SERRAGE ET DE TRANSPORT DE TYPE À ENVELOPPE DE TÊTE DE LIGNE

(30) Priority: 16.06.2020 CN 202010546781
(43) Date of publication of application: 01.06.2022
(73) Proprietor: Shanghai Shape Memory Alloy Co., Ltd., Shanghai, 201612 (CN)
(72) Inventor: WANG, Qingxin, Shanghai 201612 (CN); CHEN, Juan, Shanghai 201612 (CN); WANG, Yunbing, Shanghai 201612 (CN); PAN, Xiangbin, Shanghai 201612 (CN); WANG, Fan, Shanghai 201612 (CN); HU, Jinpeng, Shanghai 201612 (CN); ZHU, Xing, Shanghai 201612 (CN); XU, Xianchun, Shanghai 201612 (CN)
(74) Representative: J A Kemp LLP
(86) International application number: PCT/CN2021/089249
(87) International publication number: WO 2021/253980

(56) References cited:
- EP-A2- 0 698 373
- CN-A- 102 481 147
- CN-A- 102 802 538
- CN-A- 103 690 202
- CN-A- 106 618 664
- CN-A- 109 498 074
- CN-A- 111 658 026
- CN-U- 201 578 327
- CN-U- 201 578 327
- CN-U- 201 905 938
- CN-U- 212 939 808
- US-A1- 2007 112 381
- US-A1- 2007 112 381

## Description

### TECHNICAL FIELD

The present application relates to a head-end enveloping-type clamping and conveying system, in particular to an auxiliary tool for conveying a degradable occluder in the interventional treatment of congenital heart disease, belonging to the technical field of clinical medical equipment

### BACKGROUND

With the development of technology, minimally invasive treatment is more and more widely used. Compared with ordinary treatment, minimally invasive treatment has the advantages of small wound, less bleeding, fast recovery and low risk. It has attracted extensive attention and benefited the majority of patients, especially the elderly and children. In the treatment of congenital structural heart disease, interventional therapy is often used to place occluder at the lesion position, so as to achieve good curative effect. As a kind of precision medical instrument, the conveying system (i.e. push device) plays a great role in interventional surgery. Most of the traditional conveying systems are used in conjunction with metal occluder, and most of them are threaded connection or stuck connection. These structures cannot be applied to degradable occluder, or will cause great damage to degradable occluder. US 2007/0112381 A1 discloses an occlusion device and a surgical instrument and a method for its implantation/explantation.

### SUMMARY

The technical problem to be addressed in the present application is: how to make the degradable occluder have a conveying system used together therewith, so as to avoid damage to the degradable occluder.

In order to solve the above-mentioned technical problems, the technical solution of the present application is to provide a head-end enveloping-type clamping and conveying system as defined in the claims.

The present application provides a head-end enveloping-type clamping and conveying system, which can be used in conjunction with a degradable occluder to realize the minimally invasive interventional treatment of the degradable occluder, and to complete the functions of clamping, loading, conveying, locking, release, recovery, etc. of the degradable occluder. It also has good sheath passing ability and good clamping force and stability for degradable occluder. It has high firmness, and is easy to be pushed, released and conveyed, and has strong sheath passing ability and high compatibility with products. The push steel cable is internally fitted with the core shaft, which has good linkage and can transmit radial rotation for a long distance, and is suitable for the blood vessels and blood directions of different patients. The push handle, push steel cable, connecting tendon and jaw are connected in turn to realize long-distance control; In addition, it is equipped with an adjustment wire to cooperate with the jaw part, which can realize the adjustment and recovery of the occluder at the lesion position. The flexibility and safety of treatment are greatly improved and the treatment effect of patients is guaranteed by using the conveying system of the present application,

Wherein, the jaw part has two designs:

### (1) The first design is the enveloping uniform-teeth jaw

The wall of the enveloping uniform-teeth jaw is cylindrical, which can be well cooperate with the fusion ball of the degradable occluder. The enveloping-type structure has a large contact area and is not easy to pinch and damage the product; The jaw part is provided with a gap, which can cooperate with a plurality of fusion balls with similar specifications; Moreover, the degradable polymer material has a certain elasticity. The quadrangular pyramid short barb structure is used in the jaw part, which can just make the polymer material slightly concave without damage, increase the friction, make the degradable occluder more firm in the transportation process and not easy to shift and pull off.

### (2) The second design is the crocodile-tooth jaws

The head-end of the crocodile-tooth jaws has a circular opening and a smooth transition curved inner wall, so that it is not easy to scratch the fusion ball, and can make a smooth transition with the increase of force to make the clamping tighter, and has a certain margin, which can adapt to a plurality of fusion balls with similar specifications. The crocodile-tooth is at the head-end, which can firmly fix the tail of the fusion ball (the joining part with the occluder network), and can be locked left and right to prevent the displacement of the product, making it more firm in the conveying process and not easy to shift and pull off.

### BRIEF DESCRIPTION OF THE DRAWINGS

Figure 1 is a schematic diagram of the use of a head-end enveloping-type clamping and conveying system;
Figure 2 is a schematic diagram at the push handle on a head-end enveloping-type clamping and conveying system;
Figure 3 is a left view of the enveloping-type jaw;
Figure 4 is a perspective view of the enveloping-type jaw;
Figure 5 is a top view of the enveloping-type jaw;
Figure 6 is a structural schematic diagram of crocodile jaw;
Figure 7 is a left view of Figure 6.
Figure 8 is a structural schematic diagram of the connecting tendon;
Figure 9 is an internal structural schematic diagram of the connecting tendon;
Figure 10 is a structural schematic diagram of a head-end enveloping-type clamping and conveying system.

### DETAILED DESCRIPTION

In order to make the present application more obvious and easy to understand, the preferred embodiment and the accompanying drawings are described now in detail as follows.

### Embodiment 1

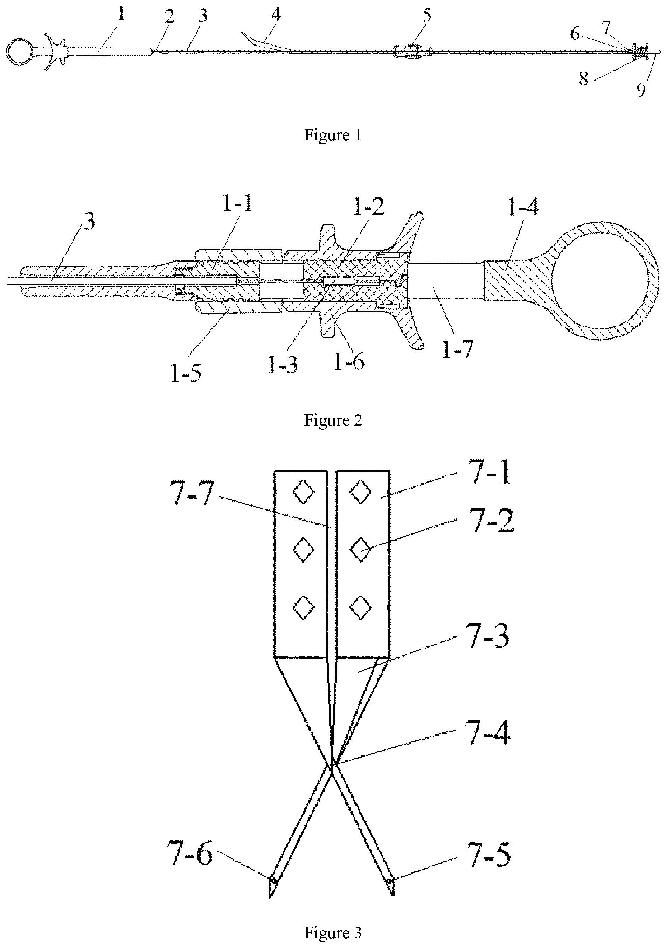

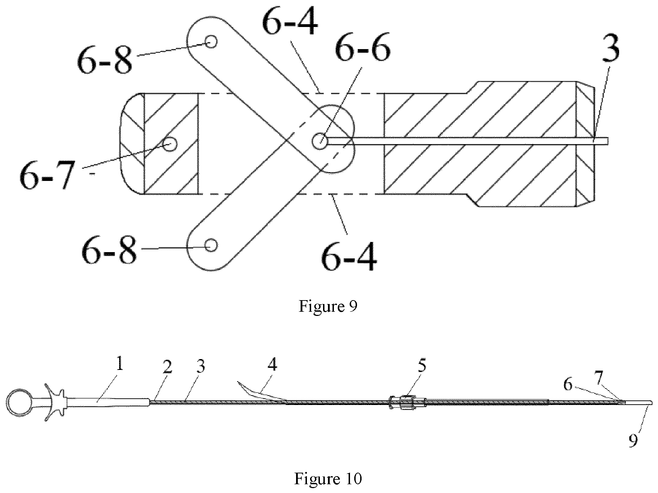

As shown in Figure 1 and Figure 10, the present application is a head-end enveloping-type clamping and conveying system, which comprises a jaw part and a pushing part, the pushing part comprises a push handle 1, a push steel cable 2, a core shaft 3, an adjustment wire, a pull wire loader 5, and a connecting tendon 6; one end of the push handle 1 is fixedly connected to one end of the push steel cable 2, the outside of the push steel cable 2 is provided with the pull wire loader 5 for pushing the push steel cable 2 into a sheath, the interior of the push handle 1 is connected to one end of the core shaft 3, and the other end of the core shaft 3 penetrates the interior of the push steel cable 2 and is connected to the connecting tendon 6, and the connecting tendon 6 is connected to the jaw part 7 for clamping and releasing an occluder. The other end of the core shaft 3 is connected to two connecting feet of the connecting tendon 6 through a first rotating shaft 6-6, the two connecting feet of the connecting tendon 6 are respectively connected to two connecting feet of the jaw part 7 for clamping and releasing the occluder through a second rotating shaft 6-8, the other end of the push steel cable 2 is fixedly connected to one end of the connecting tendon 6, the other end of the connecting tendon 6 is connected to the jaw part 7 through a third rotating shaft 6-7. The adjustment wire provided on the outside of the push steel cable 2 is provided in the pull wire loader 5. When the adjustment wire is in use, the adjustment wire cooperates with the jaw part 7 to adjust the position of the degradable occluder 8 or retract the degradable occluder 8 according to the requirements; one end of the adjustment wire connected to the degradable occluder 8 is loosed to separate the adjustment wire from the degradable occluder 8. Two ends of the adjustment wire are the other end 9 of the adjustment wire and one end 4 of the adjustment wire respectively, the other end 9 of the adjustment wire is fixed on the push handle 1; when the push steel cable 2 is provided in the sheath through the pull wire loader 5, one end 4 of the adjustment wire enters the sheath through the pull wire loader 5 and is connected to the degradable occluder 8 through the jaw part 7, and then one end 4 of the adjustment wire passes through the jaw part 7, the sheath, and the pull wire loader 5 in sequence, and returns to the push handle 1.

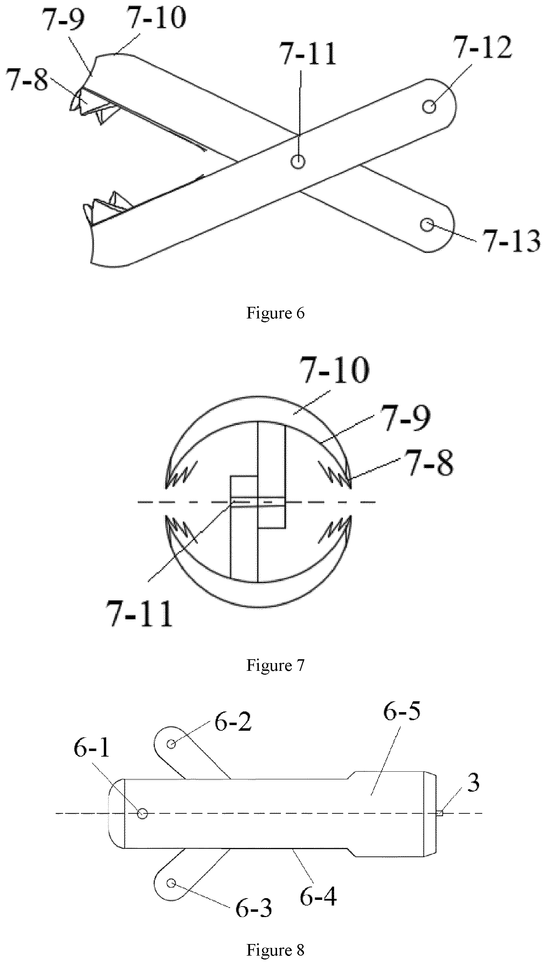

As shown in Figures 8 and 9, the connecting tendon 6 comprises a connecting tendon body 6-5, a fifth connecting foot 6-2, and a sixth connecting foot 6-3; a central hole for the core shaft 3 to pass through is provided in the connecting tendon body 6-5, both sides of the connecting tendon body 6-5 are respectively provided with a long hole 6-4 which communicates with the central hole, one end of each of the fifth connecting foot 6-2 and the sixth connecting foot 6-3 are respectively arranged on both sides of the connecting tendon body 6-5, and are connected to the two connecting feet of the jaw part 7 through the second rotating shaft 6-8, one end of each of the fifth connecting foot 6-2 and the sixth connecting foot 6-3 respectively pass through the long holes 6-4 on both sides of the connecting tendon body 6-5, and are connected to the other end of the core shaft 3 in the center hole of the connecting tendon body 6-5 through the first rotating shaft 6-6, the other end of the connecting tendon 6 is provided with a third shaft hole 6-1, the third shaft hole 6-1 of the connecting tendon 6 is connected to the jaw part 7 through the third rotating shaft 6-7.

The connecting tendon 6 cooperates with the jaw part 7 to realize long-distance transmission movement, wherein the third rotating shaft 6-7 passes through the third shaft hole 6-1 and the shaft hole on the jaw part 7, so that the two are connected as a whole. The fifth connecting foot 6-2 and the sixth connecting foot 6-3 are connected with the two connecting feet of the jaw part 7, and the ends of the fifth connecting foot 6-2 and the sixth connecting foot 6-3 are connected with the core shaft 3 and the push handle 1, so as to control their movement at the far end. In order to realize long-distance transmission, the end of the push handle 1 can control their movement.

When in use, the push handle 1 is connected to the push steel cable 2. The push steel cable 2 can be pushed into the sheath through the pull wire loader 5. Two ends of the adjustment wire are the other end 9 of the adjustment wire and one end 4 of the adjustment wire respectively, the other end 9 of the adjustment wire is fixed on the push handle 1; one end 4 of the adjustment wire enters the sheath through the pull wire loader 5 and is connected to the degradable occluder 8 through the jaw part 7, and then passes through the jaw part 7, the sheath, and the pull wire loader 5 in sequence, and returns to the push handle 1, that is, one end 4 of the adjustment wire is wrapped around for one circle and then returns to the initial position. Wherein, the adjustment wire only passes through one side of the degradable occlude 8 and penetrates on the other side, so that the adjustment wire can pull the degradable occluder 8 to move together, and the adjustment wire is not fixed with the degradable occluder 8. The push handle 1 is internally connected to the core shaft 3, which penetrates the interior of the whole push steel cable 2 and is connected to the connecting tendon 6 to control the movement of the jaw part 7, so as to clamp and release the occluder.

The core shaft 3 inside the push steel cable 2 is made of special ductile material, which can realize good linkage and radial rotation for long-distance transmission. The core shaft 3 is connected to the jaw part 7 through the connecting tendon 6, which can realize long-distance locking and release. The adjustment wire is made of special materials, which is very thin and has high strength. It is located outside the push steel cable 2 and can be withdrawn after the treatment; The adjustment wire cooperates with the jaw part 7 to be capable to adjust the position of the degradable occluder 8 according to the requirements during the treatment, which can overcome the disadvantage of being capable to be conveyed only but not being capable to be adjusted in the past, and can retract the degradable occluder 8 in case of emergency, greatly reducing the risk. It can better deal with various problems that may appear in the treatment, and make the treatment process more safe and controllable.

As shown in Figure 2, the push handle 1 comprises a wristband 1-4, a pushing head 1-3, a pushing tendon 1-2, a turnbuckle 1-5, a connecting rod 1-7, and a push-pull block 1-6. One end of the wristband 1-4 is connected to one end of the connecting rod 1-7, the other end of the connecting rod 1-7 is provided with an open threaded head 1-1, the middle of the connecting rod 1-7 is provided with an open groove, a push-pull block 1-6 that is capable to slide on the connecting rod 1-7 is sleeved on the connecting rod 1-7, one end of the core shaft 3 is fixedly connected to the pushing head 1-3 through the open threaded head 1-1, the pushing head 1-3 is fixed on the pushing tendon 1-2, the pushing tendon 1-2 is provided in the open groove, the outside of the pushing tendon 1-2 is connected to the push-pull block 1-6 sleeved on the connecting rod 1-7, the outside of the open threaded head 1-1 is sleeved with a turnbuckle 1-5 that makes the open threaded head 1-1 clamp the core shaft 3, one end of the push steel cable 2 is connected to the open threaded head 1-1.

The pushing head 1-3 is connected to the core shaft 3 through the pushing tendon 1-2, which can control the forward and backward movement of the core shaft 3, so as to remotely control the opening and closing of the jaw part 7. The turnbuckle 1-5 can be remotely locked after the jaw part 7 is closed, so as to firmly fix the occluder on the jaw part 7 of the conveying device. Wristband 1-4 are used to facilitate hand-held push and pull during operation, so as to realize its movement.

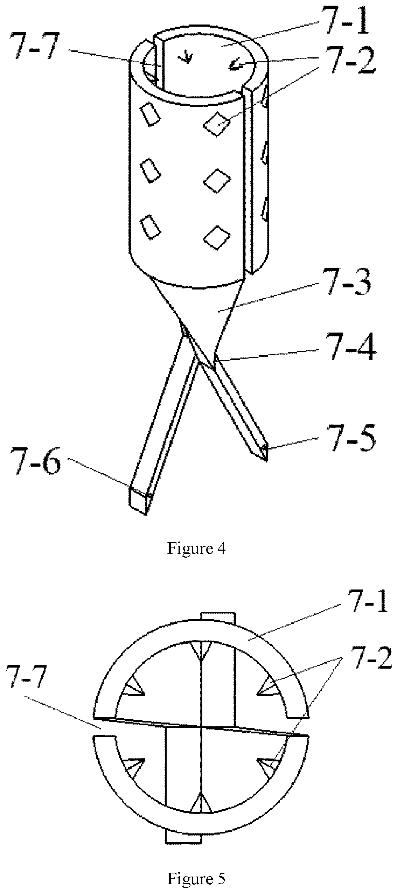

As shown in Figures 3 and 5, the jaw part 7 is an enveloping-type jaw structure comprising 18 rivets 7-2 in total located on the inner wall of the semi-cylindrical jaw 7-1, which plays a fixed anti-skid role and can limit the left, right and up, down movement of the degradable occluder 8 during use. The two semi-cylindrical jaws 7-1 combine to form an enveloping uniform-teeth jaw, the inner wall of the enveloping uniform-teeth jaw is cylindrical that is capable to cooperate well with a fusion ball of the degradable occluder 8. The enveloping-type structure has large contact area and is not easy to pinch and damage the product; the integral connector 7-3 is the integral transition part that connects the first connecting foot 7-5, the second connecting foot 7-6 and the semi-cylindrical jaw 7-1, a first shaft hole 7-4 is provided at the connection between the integral connector 7-3 and the connecting foot, the third rotating shaft 6-7 is connected to the other end of the connecting tendon 6 through the first shaft hole 7-4 on the two integral connectors 7-3, so that the jaw can open and close; a reserved gap 7-7 is provided between the two semi-cylindrical jaws 7-1, so that the jaw can clamp the product (fusion ball) and can adapt to the specifications and models of products (fusion ball) in a certain size range, that is it can cooperate with a plurality of fusion balls of similar specifications; moreover, the degradable polymer material has certain elasticity, and the inner wall of the enveloping uniform-teeth jaw uses four-pyramid short-barb structure which can just make the polymer material slightly concave without damage, increase the friction, and make the degradable occluder 8 more firm in the conveying process and not easy to shift and pull off. The first connecting foot 7-5 and the second connecting foot 7-6 connected to the third rotating shaft 6-7 are connected to the connecting foot of the connecting tendon 6, which can carry out long-distance transmission movement through the push handle 1.

### Embodiment 2

In this embodiment, as shown in Figures 6 and 7, the jaw part 7 is an alligator tooth jaw structure, the two jaws form an alligator tooth jaw, the alligator tooth jaw is composed of two curved walls 7-10 and crocodile teeth 7-8 on the curved wall 7-10, the head end of the alligator tooth jaw is a top hole 7-9 with a circular opening, and the top hole 7-9 transitions smoothly with the curved wall 7-10, which make it not easily scratch the fusion ball, and can transition smoothly with the increase of force to make the clamping tighter, and has a certain margin, which can adapt to a plurality of fusion balls of similar specifications. Crocodile teeth 7-8 surround both sides of the top hole 7-9, the curved wall 7-10 plays the role of fixing the tail end of the fusion ball (there is a boss at the connection between the tail end and the net). The top hole 7-9 can clamp the tail part of the fusion ball in the hole, and the crocodile teeth 7-8 act on the tail end of the fusion ball to fix and prevent displacement. The third rotating shaft 6-7 passes through the second shaft hole 7-11 in the middle of the two jaw bars and is connected to the other end of the connecting tendon 6, the opening and closing movement is realized by the rotation of the third rotating shaft 6-7. The third connecting foot 7-12 and the fourth connecting foot 7-13 are connected to the connecting foot of the connecting tendon 6 of the conveying device, and can carry out long-distance transmission movement through the push handle 1. Crocodile teeth 7-8 are at the head end, which can firmly fix the tail part of the fusion ball (the connecting part with the occluder network), and can be locked left and right to prevent product displacement, making it more firm in the conveying process and not easy to shift and pull off.

## Claims

1. A head-end enveloping-type clamping and conveying system, comprising a jaw part (7) and a pushing part,
wherein the pushing part comprises a push handle (1), a push steel cable (2), a core shaft (3), an adjustment wire, a pull wire loader (5), and a connecting tendon (6);
one end of the push handle (1) is fixedly connected to one end of the push steel cable (2), the outside of the push steel cable (2) is provided with the pull wire loader (5) suitable for pushing the push steel cable (2) into a sheath, the interior of the push handle (1) is connected to one end of the core shaft (3), and the other end of the core shaft (3) penetrates the interior of the push steel cable (2) and is connected to the connecting tendon (6), and the connecting tendon (6) is connected to the jaw part (7) suitable for clamping and releasing an degradable occluder (8), **characterized in that**, the jaw part (7) comprises two jaw bars, the middle positions of the two jaw bars are movably connected through a third rotating shaft (6-7), one end of each of the two jaw bars are provided with a connecting foot respectively, two connecting feet of one end of each of the two jaw bars are flexibly connected to dlw two connecting feet of the connecting tendons (6) through a further rotating shaft (6-8), the other end of each of the two jaw bars are provided with a jaw respectively; when the two connecting feet of one end of each of the two jaw bars rotate relative to each other around the third rotating shaft (6-7), the two jaws of the other end of each of the two jaw bars also rotate relative to each other, thereby clamping the degradable occluder (8); when the two connecting feet of one end of each of the two jaw bars rotate opposite to each other around the third rotating shaft (6-7), the two jaw bars of the other end of each of the two jaw bars also rotate opposite to each other, thereby releasing the degradable occluder (8), wherein, the jaw part (7) is an enveloping-type jaw structure, the two jaws are two semi-cylindrical jaws (7-1) combined to form an enveloping uniform-teeth jaw, the inner wall of the enveloping uniform-teeth jaw is a cylindrical structure that is capable to cooperate with a fusion ball of the degradable occluder (8), a plurality of rivets (7-2) for preventing the degradable occluder (8) from moving left and right or up and down are evenly distributed on the inner wall of each semi-cylindrical jaw (7-1); the jaw bar comprise the semi-cylindrical jaw (7-1), an integral connector (7-3), and the connecting foot, the integral connector (7-3) is the integral transition part that connects the connecting foot and the semi-cylindrical jaw (7-1) on the jaw bars, a first shaft hole (7-4) is provided at the connection between the integral connector (7-3) and the connecting foot on the jaw bar, the rotating shaft (6-7) is connected to the other end of the connecting tendon (6) through the first shaft hole (7-4) on the two integral connectors (7-3); a reserved gap (7-7) is provided between the two semi-cylindrical jaws (7-1); or
the jaw part (7) is a crocodile-tooth jaw structure, two jaws form crocodile-tooth jaws, the crocodile-tooth jaws consist of two curved walls (7-10) and crocodile teeth (7-8) on the curved walls (7-10), the head-end of the crocodile-tooth jaws is a top hole (7-9) with circular opening, the top hole (7-9) smoothly transitions to the curved wall (7-10), the crocodile teeth (7-8) surround both sides of the top hole (7-9), the middle positions of the two jaw bars are provided with a second shaft hole (7-11), the rotating shaft (6-7) is connected to the other end of the connecting tendon (6) through the second shaft hole (7-11) in the middle of the two jaw bars.

2. The head-end enveloping-type clamping and conveying system according to claim 1, wherein the other end of the core shaft (3) is connected to the two connecting feet of the connecting tendon (6) through a yet another rotating shaft (6-6), the other end of the push steel cable (2) is fixedly connected to one end of the connecting tendon (6), the other end of the connecting tendon (6) is connected to the jaw part (7) through the third rotating shaft (6-7)

3. The head-end enveloping-type clamping and conveying system according to claim 1 or 2, wherein the adjustment wire provided on the outside of the push steel cable (2) is provided in the pull wire loader (5).

4. The head-end enveloping-type clamping and conveying system according to claim 3, wherein two ends of the adjustment wire are another end (9) of the adjustment wire and one end (4) of the adjustment wire respectively, the other end (9) of the adjustment wire is fixed on the push handle (1); when the push steel cable (2) is provided in the sheath through the pull wire loader (5), one end (4) of the adjustment wire enters the sheath through the pull wire loader (5) and is connected to the degradable occluder (8) through the jaw part (7), and the one end (4) of the adjustment wire passes through the jaw part (7), the sheath, and the pull wire loader (5) in sequence, and returns to the push handle (1).

5. The head-end enveloping-type clamping and conveying system according to claim 2, wherein the connecting tendon (6) comprises a connecting tendon body (6-5), a fifth connecting foot (6-2), and a sixth connecting foot (6-3); wherein a central hole for the core shaft (3)to pass through is provided in the connecting tendon body (6-5), both sides of the connecting tendon body (6-5) are respectively provided with a long hole (6-4) which communicates with the central hole, one end of each of the fifth connecting foot (6-2) and the sixth connecting foot (6-3) are respectively arranged on both sides of the connecting tendon body (6-5), and are connected to the two connecting feet of the jaw part (7) through the further rotating shaft (6-8), one end of each of the fifth connecting foot (6-2) and the sixth connecting foot (6-3) respectively pass through the long holes (6-4) on both sides of the connecting tendon body (6-5), and are connected to the other end of the core shaft (3) in the center hole of the connecting tendon body (6-5) through the yet another rotating shaft (6-6), the other end of the connecting tendon (6) is provided with a third shaft hole (6-1), the third shaft hole (6-1) of the connecting tendon (6) is connected to the jaw part (7) through the rotating shaft (6-7).

6. The head-end enveloping-type clamping and conveying system according to claim 1, wherein the push handle (1) comprises a wristband (1-4), a pushing head (1-3), a pushing tendon (1-2), a turnbuckle (1-5), a connecting rod (1-7), and a push-pull block (1-6); wherein one end of the wristband (1-4) is connected to one end of the connecting rod (1-7), an opposite end of the connecting rod (1-7) is provided with an open threaded head (1-1), the middle of the connecting rod (1-7) is provided with an open groove, a push-pull block (1-6) that is capable to slide on the connecting rod (1-7) is sleeved on the connecting rod (1-7), one end of the core shaft (3) is fixedly connected to the pushing head (1-3) through the open threaded head (1-1), the pushing head (1-3) is fixed on the pushing tendon (1-2), the pushing tendon (1-2) is provided in the open groove, the outside of the pushing tendon (1-2) is connected to the push-pull block (1-6) sleeved on the connecting rod (1-7), the outside of the open threaded head (1-1) is sleeved with the turnbuckle (1-5) that makes the open threaded head (1-1) clamp the core shaft (3), one end of the push steel cable (2) is connected to the open threaded head (1-1).

## Patentansprüche

1. Kopfendenklemm- und Fördersystem der umhüllenden Art, umfassend einen Backenteil (7) und einen Schiebeteil,
wobei der Schiebeteil einen Schiebegriff (1), ein Schiebestahlseil (2), eine Kernwelle (3), einen Einstelldraht, einen Zugdrahtlader (5) und ein Verbindungsspannglied (6) umfasst;
ein Ende des Schiebegriffs (1) fixiert mit einem Ende des Schiebestahlseils (2) verbunden ist, die Außenseite des Schiebestahlseils (2) mit dem Zugdrahtlader (5) versehen ist, der geeignet ist, um das Schiebestahlseil (2) in eine Hülle zu schieben, das Innere des Schiebegriffs (1) mit einem Ende der Kernwelle (3) verbunden ist, und das andere Ende der Kernwelle (3) in das Innere des Schiebestahlseils (2) eindringt und mit dem Verbindungsspannglied (6) verbunden ist, und das Verbindungsspannglied (6) mit dem Backenteil (7) verbunden ist, der zum Klemmen und Lösen eines abbaubaren Okkluders (8) geeignet ist,
**dadurch gekennzeichnet, dass** der Backenteil (7) zwei Backenstangen umfasst, wobei die mittleren Positionen der zwei Backenstangen durch eine drehende Welle (6-7) verbunden sind, ein Ende jeder der zwei Backenstangen jeweils mit einem jeweiligen Verbindungsfuß versehen ist, zwei Verbindungsfüße eines Endes jeder der zwei Backenstangen mit zwei Verbindungsfüßen der Verbindungsspannglieder (6) durch eine weitere drehende Welle (6-8) flexibel verbunden sind, das andere Ende jeder der zwei Backenstangen mit einer jeweiligen Backe versehen sind; wenn sich die zwei Verbindungsfüße eines Endes jeder der zwei Backenstangen relativ zueinander um die drehende Welle (6-7) drehen, sich die zwei Backen des anderen Endes jeder der zwei Backenstangen ebenso relativ zueinander drehen, wodurch der abbaubare Okkluder (8) eingeklemmt wird; wenn sich die zwei Verbindungsfüße eines Endes jeder der zwei Backenstangen entgegengesetzt zueinander um die drehende Welle (6-7) drehen, sich die zwei Backenstangen des anderen Endes jeder der zwei Backenstangen ebenso entgegengesetzt zueinander drehen, wodurch der abbaubare Verschluss (8) gelöst wird, wobei der Backenteil (7) eine Backenstruktur der umhüllenden Art ist, die zwei Backenteile zwei halbzylindrische Backenteile (7-1) sind, die kombiniert sind, um einen umhüllenden Backenteil mit gleichmäßiger Verzahnung auszubilden, die Innenwand des umhüllenden Backenteils mit gleichmäßiger Verzahnung eine zylindrische Struktur ist, die in der Lage ist, mit einer Schmelzkugel des abbaubaren Okkluders (8) zusammenzuwirken, eine Mehrzahl von Nieten (7-2), die verhindern, dass sich der abbaubare Okkluder (8) nach links und rechts oder nach oben und unten bewegt, gleichmäßig auf der Innenwand jedes halbzylindrischen Backenteils (7-1) verteilt sind; die Backenstange die halbzylindrische Backe (7-1), einen integralen Verbinder (7-3) und den Verbindungsfuß umfasst, der integrale Verbinder (7-3) der integrale Übergangsteil ist, der den Verbindungsfuß und die halbzylindrische Backe (7-1) an den Backenstangen verbindet, ein erstes Wellenloch (7-4) an der Verbindung zwischen dem integralen Verbinder (7-3) und dem Verbindungsfuß an der Backenstange bereitgestellt ist, die drehende Welle (6-7) mit dem anderen Ende des Verbindungsspannglied (6) durch das erste Wellenloch (7-4) an den zwei integralen Verbindern (7-3) verbunden ist; ein reservierter Spalt (7-7) zwischen den zwei halbzylindrischen Backen (7-1) bereitgestellt ist; oder
der Backenteil (7) eine Krokodilzahnbackenstruktur ist, zwei Backen Krokodilzahnbacken ausbilden, die Krokodilzahnbacken aus zwei gebogenen Wänden (7-10) und Krokodilzähnen (7-8) auf den gebogenen Wänden (7-10) bestehen, das Kopfende der Krokodilzahnbacken ein oberes Loch (7-9) mit kreisförmiger Öffnung ist, das obere Loch (7-9) fließend in die gebogene Wand (7-10) übergeht, die Krokodilzähne (7-8) beide Seiten des oberen Lochs (7-9) umgeben, die mittleren Positionen der zwei Backenstangen mit einem zweiten Wellenloch (7-11) versehen sind, die drehende Welle (6-7) mit dem anderen Ende des Verbindungsspannglieds (6) durch das zweite Wellenloch (7-11) in der Mitte der zwei Backenstangen verbunden ist.

2. Kopfendenklemm- und Fördersystem der umhüllenden Art nach Anspruch 1, wobei das andere Ende der Kernwelle (3) mit den zwei Verbindungsfüßen des Verbindungsspannglieds (6) durch noch eine andere drehenden Welle (6-6) verbunden ist, das andere Ende des Schiebestahlseils (2) mit einem Ende des Verbindungsspannglieds (6) fixiert verbunden ist, das andere Ende des Verbindungsspannglieds (6) mit dem Backenteil (7) durch die drehende Welle (6-7) verbunden ist.

3. Kopfendenklemm- und Fördersystem der umhüllenden Art nach Anspruch 1 oder 2, wobei der Anpassungsdraht, der auf der Außenseite des Schiebestahlseils (2) bereitgestellt ist, in dem Zugdrahtlader (5) bereitgestellt ist.

4. Kopfendenklemm- und Fördersystem der umhüllenden Art nach Anspruch 3, wobei zwei Enden des Anpassungsdrahts ein anderes Ende (9) des Anpassungsdrahts beziehungsweise ein Ende (4) des Anpassungsdrahts sind, das andere Ende (9) des Anpassungsdrahts auf dem Schiebegriff (1) fixiert ist; wenn das Schiebestahlseil (2) in der Hülle durch den Zugdrahtlader (5) bereitgestellt ist, ein Ende (4) des Anpassungsdrahts in die Hülle durch den Zugdrahtlader (5) eintritt und durch den Backenteil (7) mit dem abbaubaren Okkluder (8) verbunden ist, und das eine Ende (4) des Anpassungsdrahts durch den Backenteil (7), die Hülle und den Zugdrahtlader (5) in der Folge passiert und zu dem Schiebegriff (1) zurückkehrt.

5. Kopfendenklemm- und Fördersystem der umhüllenden Art nach Anspruch 2, wobei das Verbindungsspannglied (6) einen Verbindungsspanngliedkörper (6-5), einen fünften Verbindungsfuß (6-2) und einen sechsten Verbindungsfuß (6-3) umfasst;
wobei ein zentrales Loch für den Durchgang der Kernwelle (3) in dem Verbindungsspanngliedkörper (6-5) bereitgestellt ist, beide Seiten des Verbindungsspanngliedkörpers (6-5) jeweils mit einem langen Loch (6-4) versehen sind, das mit dem zentralen Loch in Verbindung steht, ein Ende jedes des fünften Verbindungsfußes (6-2) und des sechsten Verbindungsfußes (6-3) jeweils auf beiden Seiten des Verbindungsspanngliedkörpers (6-5) angeordnet und mit den zwei Verbindungsfüßen des Backenteils (7) durch die weitere drehende Welle (6-8) verbunden sind, ein Ende jedes des fünften Verbindungsfußes (6-2) beziehungsweise des sechsten Verbindungsfußes (6-3) durch die langen Löcher (6-4) auf beiden Seiten des Verbindungsspanngliedkörpers (6-5) passiert und mit dem anderen Ende der Kernwelle (3) in dem zentralen des Verbindungsspanngliedkörpers (6-5) durch noch eine weitere drehende Welle (6-6) verbunden ist, das andere Ende des Verbindungsspannglieds (6) mit einem dritten Wellenloch (6-1) versehen ist, das dritte Wellenloch (6-1) des Verbindungsspannglieds (6) mit dem Backenteil (7) durch die drehende Welle (6-7) verbunden ist.

6. Kopfendenklemm- und Fördersystem der umhüllenden Art nach Anspruch 1, wobei der Schiebegriff (1) ein Handgelenkband (1-4), einen Schiebekopf (1-3), ein Schiebespannglied (1-2), ein Spannschloss (1-5), eine Verbindungsstange (1-7) und einen Schiebe-Zug-Block (1-6) umfasst;
wobei ein Ende des Armbands (1-4) mit einem Ende der Verbindungsstange (1-7) verbunden ist, ein gegenüberliegendes Ende der Verbindungsstange (1-7) mit einem offenen Gewindekopf (1-1) versehen ist, die Mitte der Verbindungsstange (1-7) mit einer offenen Rille versehen ist, ein Schiebe-Zug-Block (1-6), der auf der Verbindungsstange (1-7) gleiten kann, auf der Verbindungsstange (1-7) aufgeschoben ist, ein Ende der Kernwelle (3) durch den offenen Gewindekopf (1-1) mit dem Schiebekopf (1-3) fixiert verbunden ist, der Schiebekopf (1-3) an dem Schiebespannglied (1-2) fixiert ist, das Schiebespannglied (1-2) in der offenen Rille bereitgestellt ist, die Außenseite des Schiebespannglieds (1-2) mit dem Schiebe-Zug-Block (1-6) verbunden ist, der auf die Verbindungsstange (1-7) aufgeschoben ist, die Außenseite des offenen Gewindekopfes (1-1) auf das Spannschloss (1-5) aufgeschoben ist, das den offenen Gewindekopf (1-1) an der Kernwelle (3) einklemmt, ein Ende des Schiebestahlseils (2) mit dem offenen Gewindekopf (1-1) verbunden ist.

## Revendications

1. Système de serrage et de transport de type enveloppant en extrémité de tête, comprenant une partie mâchoire (7) et une partie poussante,
dans lequel la partie poussante comprend une poignée de poussée (1), un câble d'acier de poussée (2), une tige âme (3), un fil d'ajustement, un chargeur de fil de tirage (5), et un câble de liaison (6) ;
une extrémité de la poignée de poussée (1) est reliée de façon fixe à une extrémité du câble d'acier de poussée (2), l'extérieur du câble d'acier de poussée (2) est pourvu du chargeur de fil de tirage (5) approprié pour pousser le câble d'acier de poussée (2) dans une gaine, l'intérieur de la poignée de poussée (1) est relié à une extrémité de la tige âme (3), et l'autre extrémité de la tige âme (3) pénètre à l'intérieur du câble d'acier de poussée (2) et est reliée au câble de liaison (6), et le câble de liaison (6) est relié à la partie mâchoire (7) appropriée pour serrer et relâcher un obturateur dégradable (8),
**caractérisé en ce que** la partie mâchoire (7) comprend deux barres de mâchoire, les positions médianes des deux barres de mâchoire sont reliées de façon mobile par l'intermédiaire d'une tige rotative (6-7), une extrémité de chacune des deux barres de mâchoire est pourvue d'un pied de liaison, respectivement, deux pieds de liaison d'une extrémité de chacune des deux barres de mâchoire sont reliés de façon flexible aux deux pieds de liaison des câbles de liaison (6) par l'intermédiaire d'une tige rotative supplémentaire (6-8), l'autre extrémité de chacune des deux barres de mâchoire est pourvue d'une mâchoire, respectivement ; lorsque les deux pieds de liaison d'une extrémité de chacune des deux barres de mâchoire entrent en rotation l'un relativement à l'autre autour de la tige rotative (6-7), les deux mâchoires de l'autre extrémité de chacune des deux barres de mâchoire entrent également en rotation l'une relativement l'autre, ainsi serrant l'obturateur dégradable (8) ; lorsque les deux pieds de liaison d'une extrémité de chacune des deux barres de mâchoire entrent en rotation de façon opposée l'un à l'autre autour de la tige rotative (6-7), les deux barres de mâchoire de l'autre extrémité de chacune des deux barres de mâchoire entrent également en rotation de façon opposée l'une à l'autre, ainsi relâchant l'obturateur dégradable (8),
dans lequel la partie mâchoire (7) est une structure de mâchoire de type enveloppant, les deux mâchoires sont deux mâchoires semi-cylindriques (7-1) combinées pour former une mâchoire enveloppante à dents uniformes, la paroi intérieure de la mâchoire enveloppante à dents uniformes est une structure cylindrique qui est capable de coopérer avec une bille de fusion de l'obturateur dégradable (8), une pluralité de rivets (7-2) pour empêcher l'obturateur dégradable (8) d'entrer en mouvement vers la gauche et la droite ou vers le haut et le bas sont uniformément distribués sur la paroi intérieure de chaque mâchoire semi-cylindrique (7-1) ; la barre de mâchoire comprend la mâchoire semi-cylindrique (7-1), une pièce de liaison monobloc (7-3), et le pied de liaison, la pièce de liaison monobloc (7-3) est la partie de transition monobloc qui relie le pied de liaison et la mâchoire semi-cylindrique (7-1) sur les barres de mâchoire, un premier trou de tige (7-4) est prévu au niveau de la liaison entre la pièce de liaison monobloc (7-3) et le pied de liaison sur la barre de mâchoire, la tige rotative (6-7) est reliée à l'autre extrémité du câble de liaison (6) par l'intermédiaire du premier trou de tige (7-4) sur les deux pièces de liaison monoblocs (7-3) ; un espace inversé (7-7) est prévu entre les deux mâchoires semi-cylindriques (7-1) ; ou
la partie mâchoire (7) est une structure de mâchoires à dents de crocodile, deux mâchoires forment des mâchoires à dents de crocodile, les mâchoires à dents de crocodile sont constituées de deux parois incurvées (7-10) et de dents de crocodile (7-8) sur les parois incurvées (7-10), l'extrémité de tête des mâchoires à dents de crocodile est un trou supérieur (7-9) avec une ouverture circulaire, la transition entre le trou supérieur (7-9) et la paroi incurvée (7-10) est homogène, les dents de crocodile (7-8) entourent les deux côtés du trou supérieur (7-9), les positions médianes des deux barres de mâchoire sont pourvues d'un deuxième trou de tige (7-11), la tige rotative (6-7) est reliée à l'autre extrémité du câble de liaison (6) par l'intermédiaire du deuxième trou de tige (7-11) dans le milieu des deux barres de mâchoire.

2. Système de serrage et de transport de type enveloppant en extrémité de tête selon la revendication 1, dans lequel l'autre extrémité de la tige âme (3) est reliée à deux pieds de liaison du câble de liaison (6) par l'intermédiaire d'une tige rotative additionnelle (6-6), l'autre extrémité du câble d'acier de poussée (2) est reliée de façon fixe à une extrémité du câble de liaison (6), l'autre extrémité du câble de liaison (6) est reliée à la partie mâchoire (7) par l'intermédiaire de la tige rotative (6-7).

3. Système de serrage et de transport de type enveloppant en extrémité de tête selon la revendication 1 ou 2, dans lequel le fil d'ajustement prévu sur l'extérieur du câble d'acier de poussée (2) est prévu dans le chargeur de fil de tirage (5).

4. Système de serrage et de transport de type enveloppant en extrémité de tête selon la revendication 3, dans lequel deux extrémités du fil d'ajustement sont une autre extrémité (9) du fil d'ajustement et une extrémité (4) du fil d'ajustement respectivement, l'autre extrémité (9) du fil d'ajustement est fixée sur la poignée de poussée (1) ; lorsque le câble d'acier de poussée (2) est prévu dans la gaine par l'intermédiaire du chargeur de fil de tirage (5), une extrémité (4) du fil d'ajustement entre dans la gaine par l'intermédiaire du chargeur de fil de tirage (5) et est reliée à l'obturateur dégradable (8) par l'intermédiaire de la partie mâchoire (7), et l'une extrémité (4) du fil d'ajustement passe à travers la partie mâchoire (7), la gaine, et le chargeur de fil de tirage (5) en séquence, et retourne à la poignée de poussée (1).

5. Système de serrage et de transport de type enveloppant en extrémité de tête selon la revendication 2, dans lequel le câble de liaison (6) comprend un corps de câble de liaison (6-5), un cinquième pied de liaison (6-2), et un sixième pied de liaison (6-3) ;
dans lequel un trou central pour que la tige âme (3) passe à travers est prévu dans le corps de câble de liaison (6-5), les deux côtés du corps de câble de liaison (6-5) sont respectivement pourvus d'un trou long (6-4) qui communique avec le trou central, une extrémité de chacun du cinquième pied de liaison (6-2) et du sixième pied de liaison (6-3) est respectivement agencée sur les deux côtés du corps de câble de liaison (6-5), et est reliée aux deux pieds de liaison de la partie mâchoire (7) par l'intermédiaire de la tige rotative supplémentaire (6-8), une extrémité de chacun du cinquième pied de liaison (6-2) et du sixième pied de liaison (6-3) passe respectivement à travers les trous longs (6-4) sur les deux côtés du corps de câble de liaison (6-5), et est reliée à l'autre extrémité de la tige âme (3) dans le trou central du corps de câble de liaison (6-5) par l'intermédiaire de la tige rotative additionnelle (6-6), l'autre extrémité du câble de liaison (6) est pourvue d'un troisième trou de tige (6-1), le troisième trou de tige (6-1) du câble de liaison (6) est relié à la partie mâchoire (7) par l'intermédiaire de la tige rotative (6-7).

6. Système de serrage et de transport de type enveloppant en extrémité de tête selon la revendication 1, dans lequel la poignée de poussée (1) comprend un bracelet (1-4), une tête poussante (1-3), un câble poussant (1-2), un tendeur à vis (1-5), une tringle de liaison (1-7), et un bloc de poussée-tirage (1-6),
dans lequel une extrémité du bracelet (1-4) est reliée à une extrémité de la tringle de liaison (1-7), une extrémité opposée de la tringle de liaison (1-7) est pourvue d'une tête filetée ouverte (1-1), le milieu de la tringle de liaison (1-7) est pourvu d'une rainure ouverte, un bloc de poussée-tirage (1-6) qui est capable de coulisser sur la tringle de liaison (1-7) est emmanché sur la tringle de liaison (1-7), une extrémité de la tige âme (3) est reliée de façon fixe à la tête poussante (1-3) par l'intermédiaire de la tête filetée ouverte (1-1), la tête poussante (1-3) est fixée sur le câble poussant (1-2), le câble poussant (1-2) est prévu dans la rainure ouverte, l'extérieur du câble poussant (1-2) est relié au bloc de poussée-tirage (1-6) emmanché sur la tringle de liaison (1-7), le tendeur à vis (1-5), qui force la tête filetée ouverte (1-1) à serrer la tige âme (3), est emmanché sur l'extérieur de la tête filetée ouverte (1-1), une extrémité du câble d'acier de poussée (2) est reliée à la tête filetée ouverte (1-1).
